(19)

Europäisches
Patentamt
European
Patent Office
Office européen
des brevets

(11) **EP 3 827 852 A1**

(12) **EUROPEAN PATENT APPLICATION**

(43) Date of publication:
**02.06.2021  Bulletin 2021/22**

(51) Int Cl.:
**A61M 1/10** *(2006.01)*     **A61M 1/12** *(2006.01)*

(21) Application number: **19211940.2**

(22) Date of filing: **27.11.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(71) Applicant: **Berlin Heart GmbH**
**12247 Berlin (DE)**

(72) Inventors:
• **Peters, Oliver**
**14129 Berlin (DE)**

• **Bykov, Valentin**
**12249 Berlin (DE)**
• **Richert, Hendryk**
**12487 Berlin (DE)**
• **Frischke, Michael**
**15834 Rangsdorf (DE)**

(74) Representative: **Pfenning, Meinig & Partner mbB**
**Patent- und Rechtsanwälte**
**Joachimsthaler Straße 10-12**
**10719 Berlin (DE)**

(54) **BLOOD PUMP WITH MAGNETICALLY LEVITATED ROTOR**

(57)     The application pertains to a blood pump system, in particular a ventricular assist device, VAD, the system including a blood pump, which comprises: a housing, including an inlet and an outlet, preferably an axial influx and a tangential outflow; a motor actuator, wherein the motor includes a plurality of motor coils (for driving an impeller); an impeller, wherein the impeller is located in the housing and includes a plurality of rotor magnets.

The system further comprises a drive line; and a control unit for controlling operation of the pump, the control unit configured to:
- operate the motor, such that the impeller rotates around an axis; and
- measure the rotor position in a direction along the axis using at least one of the plurality of the motor coils.

Figure 1

EP 3 827 852 A1

**Description**

Background

The ventricular assist market offers currently no good implantable heart assist solution for children with a body surface area below 103m$^2$. Large VADs, intended and approved for the use in adults, are sometimes used in children due to the lack of a more suitable implantable VAD. The outcome of this off-label use of adult VADs in children is significantly worse than the approved therapy in adults.

To adapt a VAD for children, the size and fluidic performance have to be optimized without compromising the hemocompatibility.

Magnetically levitated pumps are currently regarded as state of the art to implement a hemocompatible blood pump. However, the miniaturization of a magnetic bearing is limited by its complexity and number of components. This patent application provides a novel solution to reduce the complexity and number of components in a magnetically beared blood pump.

Summary of the Invention

The pumps, motor drivers and methods described herein allow operation of a motor for driving the rotor, measuring the rotor position using the motor and influencing the rotor position. Further embodiments are described in the appended claims and can be understood from the summary and the detailed description given below.

To use the motor for driving the rotor and for measuring the rotor position in at least one degree of freedom (DOF), such as, for example, along a direction of the axis of rotation, several embodiments and improvements over the known prior art are suggested.

In a first aspect, a control unit for controlling a VAD is configured to reduce or eliminate switching noise.

Description of the Drawings

Figure 1 shows a concept of a magnetically levitated ventricular assist device;

Figure2 shows a blood pump in which two axial flux motors are used to compensate reluctance forces (Source: US 2016/0281728A1);

Figure 3 shows a VAD with a radial motor which balances the radial reluctance forces between rotor and stator (Source: US5588812A);

Figure 4 shows a pump with compensation of reluctance forces by using a magnetic compensation bearing (Source: US2016/0281728A1);

Figure 5 shows a VAD with ironless axial flux motor (Source: US006071093A)

Figure 6 shows the principles of simultaneous axial force and torque generation with an axial flux motor (Source: US006071093A);

Figure 7 shows the typical current and voltage waveforms for the self-sensing operation according to US6302661B1;

Figure 8 shows a block diagram of the rotor position estimation as disclosed in US6302661B1;

Figure 9 shows the block diagram of a VAD with sensorless magnetically levitation according to the state of the art;

Figure 10 shows the block diagram of a VAD with sensorless magnetically levitation with additional components that are improving system redundancy and rotor position measurement signal-to-noise ratio;

Figure 11 shows the electrical model of a direct current motor;

Figure 12 shows the electrical structure of a brushless direct current motor;

Figure 13 shows the electrical and mathemematical structure of state of the art electromotoric force measurements systems as used for sensorless commutation of BLDC motors;

Figure 14 shows the method of using an inductive shunt to measure the BEMF;

Figure 15 shows an analog BEMF replication

Figure 16 shows the current sampling using a combined resistive and inductive shunt;

Figure 17 shows a method to emulate an inductive shunt with an inductive shunt and filtering;

Figure 18 shows the filter structure used to generate magnetic field strength measurement from the BEMF measurement that uses a matched high pass low pass filter to create a flat frequency response;

Figure 19 shows an arrangement of actuator coils that can be used to measure the axial and rotational position of the rotor with eddy current measurements through the actuator coils;

Figure 20 shows an equivalent circuit diagram of an actuator coil with eddy current measurement and capacitive tuning;

Figure 21 shows various implementations of a magnetically sensitive tuning capacitor;

Figure 22 shows how a tuning network can be used to modify the resonant frequency of the actuator coil with dedicated sensor elements with voltage signal output;

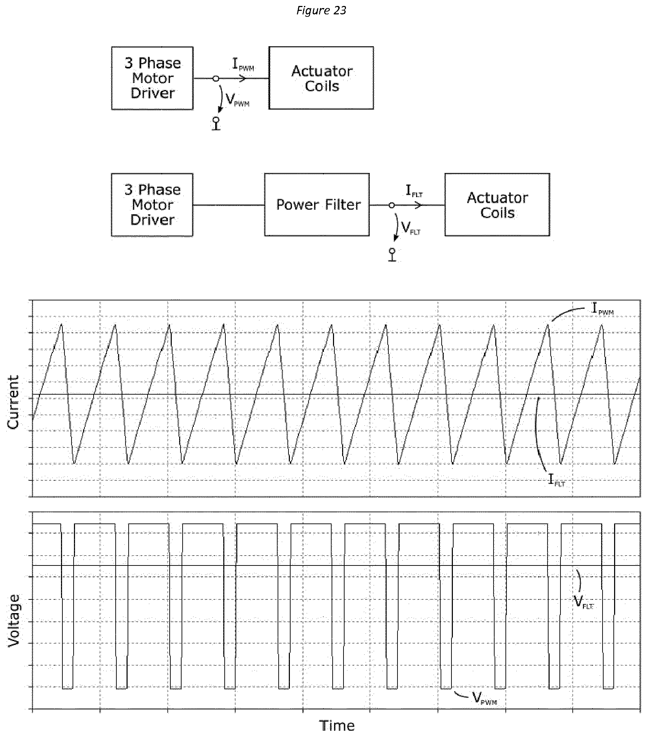

Figure 23 shows the voltage and current waveforms in motor drivers with and without power filters;

Figure 24 shows the energy flow and spectrum on the driveline when connecting a RF source to the driveline wires;

Figure 25 shows the energy flow and spectrum for the a controller and pump system with integrated RF actuator coil impedance measurement and RF filters;

Figure 26 shows a block diagram of noise filters in the current measurements chain;

Figure 27 shows a block diagram of a class AB motor driver with a dynamic power supply

Figure 28 shows an ac-inverter motor controller;

Figure 29 shows the spectrum of motor driver PWM signal and RF impedance measurement without the influence of jitter;

Figure 30 shows the influence of clock jitter on the PWM spectrum and the RF impedance measurement spectrum;

Figure 31 shows a block diagram of a motor driver and impedance measurement with shared clock source;

Figure 32 shows the electrical structure of a power connector with additional sense contacts

Figure 33 illustrates a motor connection scheme that uses a fourth driveline wire to increase VAD system redundancy;

Figure 34 illustrates a motor connection scheme that connects a dedicated bearing coil to the star point and a fourth driveline wire;

Figure 35 illustrates a motor connection scheme with six driveline wires, a motor and a dedicated bearing coil where every driveline can fail with an open connection and pump operation is still possible;

Figure 36 illustrates a motor connection scheme with four driveline wires, a motor and a dedicated bearing coil without fail safe features;

Figure 37 illustrates a motor connection scheme with seven driveline wires, a motor and a dedicated bearing coil is which even after a driveline open connection failure the pump can be operated without reduced motor efficiency;.

Figure 38 shows a cross-section of an embodiment of a blood pump to be used as a VAD that uses a combined rotational and linear motor;

Figures 39 a and b show a) a cross-section of an embodiment of a blood pump to be used as a VAD according to this document and a b) top view of a section of the rotor of the blood pump;

Figure 40 shows a cross-section of an embodiment of a blood pump to be used as a VAD according to this document;

Figure 41 shows a cross-section of an embodiment of a blood pump to be used as a VAD according to this document;

Figure 42 shows a cross-section of an embodiment of a blood pump to be used as a VAD according to this document;

Figure 43 shows a cross-section of an embodiment of a blood pump to be used as a VAD according to this document; and

Figure 44 shows a cross-section of an embodiment of a blood pump to be used as a VAD according to this document.

Detailed Description

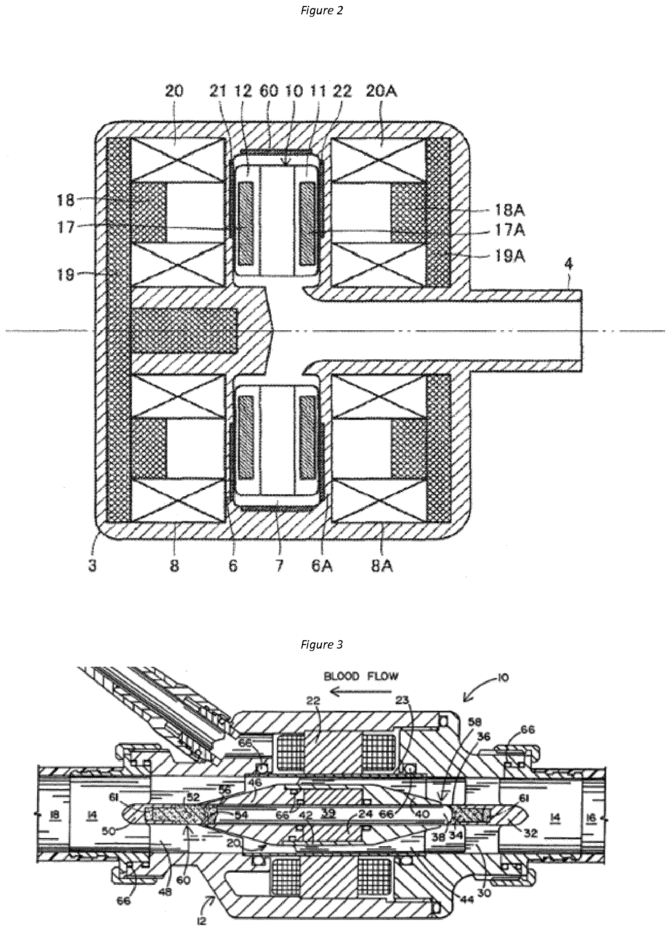

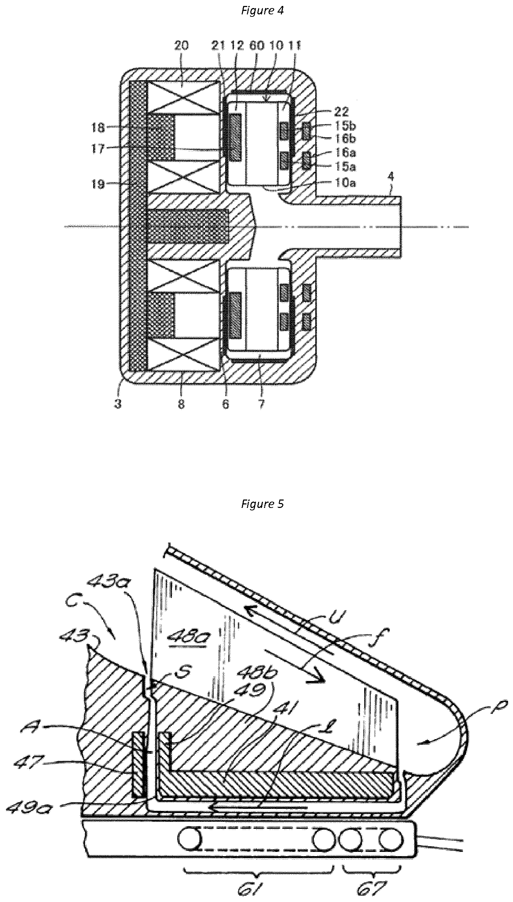

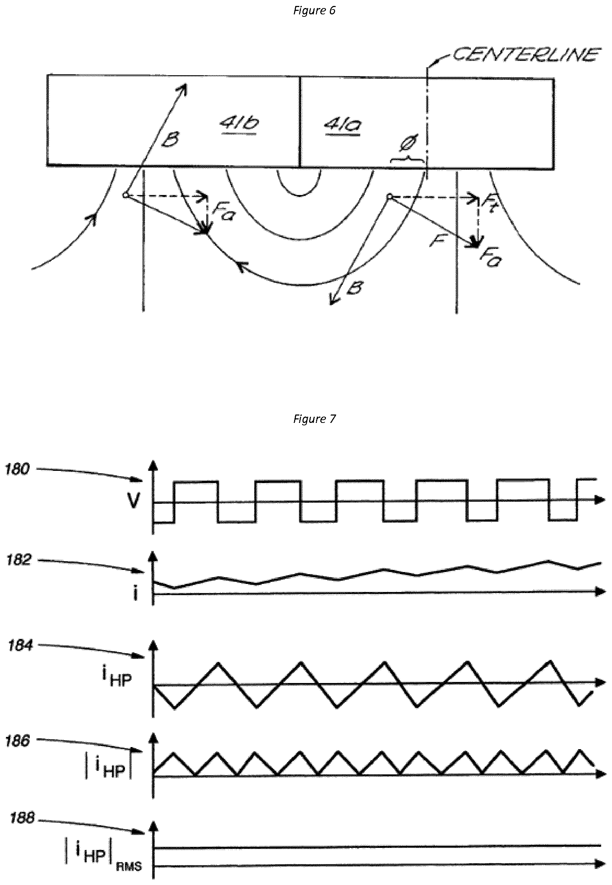

To levitate a rotor, all forces acting on the rotor have to be counteracted by equal opposing forces. A common method uses passive magnets to create counter-forces when the rotor is displaced.
According to Earnshaw's theorem, not all degrees of freedom can be stabilized using passive magnets. A magnetic arrangement that stabilizes a certain axis will always destabilize another axis by the same amount.
Earnshaw's theorem also states that the sum of all stabilities and instabilities is always zero for arrangements of passive magnets. The common approach is to stabilize some axis using magnets and create some instable degrees of freedom (DOF) which have points of instable equilibria. The DOF with an instable equilibrium are then actively controlled. A common control strategy is zero force control which balances the DOF at the instable equilibrium to minimize the necessary power for levitation.
At the zero force position or instable equilibrium, the power consumption can approach zero and is only limited by the noise and time delays in the control loop. Common for implantable blood pumps are power consumptions of 500mW (e.g. Berlin Heart Incor) for the levitation control.
When the zero force control is not in operation, then the rotor leaves the equilibrium point and is accelerated until it makes contact with the pump housing. The force acting on the rotor when it is resting on the pump housing is equal to the maximum force the rotor can tolerate under zero force control. This force is also equal to the force necessary to detach the rotor from the pump housing. Therefore, lowering the detachment force is only possible when also reducing the maximum tolerable load on the rotor. The peak detachment power can be orders of magnitude higher than the zero force levitation power. In the INCOR VAD it is three orders magnitude (0.5W for levitation and 500W for detachment) and in the Heart Mate 3 VAD it is roughly two orders of magnitude (0.5W for levitation and 50W for detachment). Reducing the detachment forces is one way of reducing the detachment power.
The force between rotor magnets and motor stator, usually reluctance forces, can also contribute significantly to the load on the rotor as well as the detachment forces. Most brushless direct current (BLDC) motors have a magnetic rotor and iron in the stator. This leads to constant attraction forces between rotor and the stator. This load force can be reduced by using two stator components whose forces on the rotor are cancelling each other out, as can be seen in the stator configuration in Figure 2 (US20160281728A1) and Figure 4 (US20160281728A1.) Another option to balance the static reluctance forces is to use a radial flux motor like it can be found in the Heart Mate 2 VAD (US5588812A, Figure 3). However, while the load force can be balanced, the magnetic stiffness is always influenced negatively by introducing more iron as part of the motor stators. This leads to disturbance forces when the rotor is not perfectly balanced at its equilibrium position.
The reluctance load force can be eliminated completely by using an ironless stator configuration. The ironless stator also has zero influence on the magnetic suspension stiffness of the rotor.
An ironless axial flux disc motor can generate torque as well as positive and negative pull force. The patent document US6071093A (Figure 5, Figure 6) shows a radial blood flow pump with an ironless axial flux motor where the motor coils are used for axial thrust generation and torque generation.
The ironless (coreless and yokeless) axial flux motor is so far not used in a VAD system. The reason is probably the reduced motor efficiency of an ironless motor compared to a motor with iron yoke or cores. However, in an actively levitated VAD system the drawback of lower efficiency opposes the gain in levitation stability. As long as the motor can be cooled sufficiently without warming the blood too much, the ironless or iron reduced axial flux motor can lead to a smaller VAD device because a yoke or magnetic compensation bearing are no longer necessary. A thermal management which implements the features presented in the patent application EP19159286.4 can make the ironless axial flux motor feasible for use in an implantable VAD.

The pump presented in the patent document US6071093A uses dedicated sensors to detect the axial rotor position. These sensors have to be read out by additional driveline wires or an electronic system which is integrated into the pump. Both solution increase the size of the implantable VAD system and therefore reduce the viability for a pediatric VAD system.

Sensorless operation of BLDC motors is commonly used in VAD systems. Sensorless BLDC operation refers commonly to a sensorless commutation of the motor phases by measuring the induced back electromotive force (BEMF) or estimating the BEMF by measuring the current in the motor phases and estimating the BEMF based on the current waveforms. The BEMF signal is proportional to rotor speed. This implies that the signal is low at low rotational speeds and zero at standstill. Below a certain speed the noise of the current or voltage measurement compromises the BEMF detection and sensorless BEMF commutation is not possible.

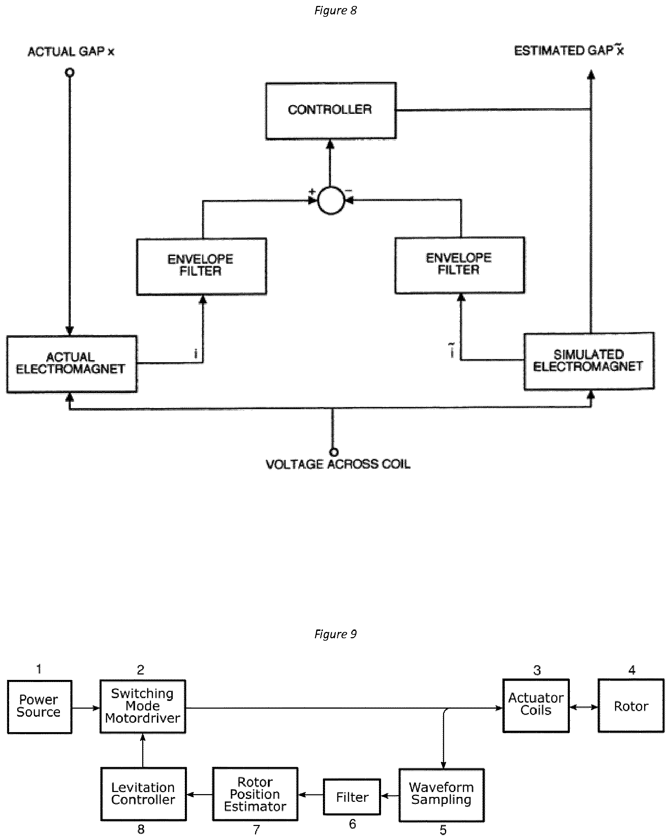

For commutation and motor operation only the rotational position of the rotor has to be known and measured. For magnetic levitation the position of the rotor in the instable DOF, which is usually a linear DOF, has to be measured. The patent document US6302661B1 describes a method to generate an electronic signal related to rotor position in a linear DOF through a combination of current and voltage provided by the activating coils. The voltage and current waveform (as shown in the patent document US6302661B1) to be measureable in the activating coils are displayed in Figure 7. The intended method (see Figure 8; also taken from US6302661B1) to extract the rotor position, in this case the bearing gap, uses an envelope filter on the current measurements to condition the signals from the sensors and uses a control loop with a simulation model of the electromagnet to estimate the bearing gap.

Efforts to replicate the presented rotor position measurement described in US6302661B1 have shown that a rotor position signal is in fact detectable. However, the signal noise and measurement delay have to meet certain requirements to be able to levitate a rotor based on the measurement. Both requirements are more demanding for a smaller and lighter rotor. Therefore, the requirements for levitation of an implantable pediatric VAD system are especially hard to meet. The measurement methods described in US6302661B1 generate too much noise. The presented invention discloses novel methods to reduce the measurement noise at various stages in the measurement chain.

One of the biggest contributions to noise originates from the switching events in the motor driver. The switching is clearly visible in the voltage and current waveforms (see for example Figure 7).

According to the invention, switching noise from the motor driver can either be reduced or eliminated at the source by changing the motor driver, be isolated from the measurement hardware with filters or the measurement hardware or method could be made insensitive to the switching noise. The main noise source in a state of the art VAD motor driver (Figure 9) is the switching mode motor driver 2.

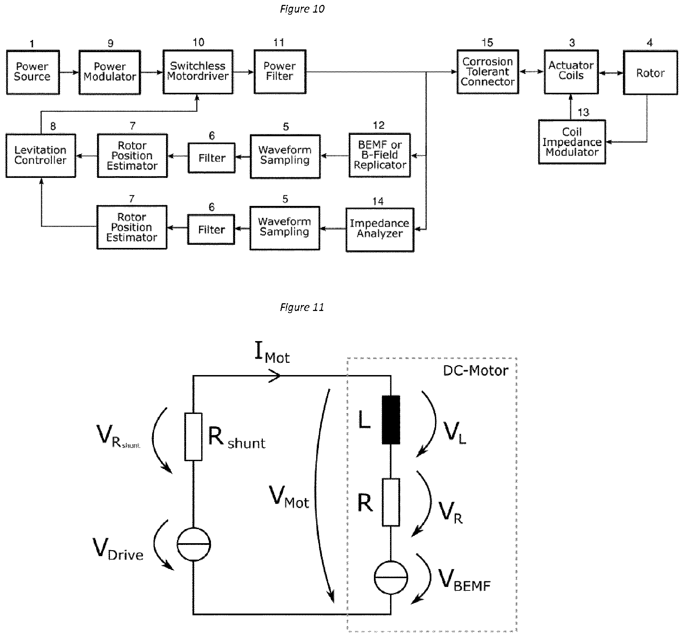

According to one aspect of the invention as shown in Figure 10, the switching noise can be significantly reduced with a switchless motor driver 10, which uses class AB power stages instead of class D PWM power stages, even though Class AB efficiency is lower than the efficiency of a PWM power stage. Class AB efficiency is 78.5% at its maximum at peak power output and reduces with lower output power. By inserting an optional power modulator 9 between power source 1 and the switchless motor driver 10, the peak efficiency can be accomplished at any operating point. Alternatively, three (or more) power modulators, e.g. tracking DC-DC-converters or DC-AC-converters can be used as a low noise motor driver replacing the switchless motor driver 10 in Figure 10. Optionally, the remaining noise left at the output terminals of the motor driver 10 is filtered with a three phase power filter 11, which suppresses the PWM frequency and its overtones in voltage and current. The voltage and current signals at the output of the power filter 11 are mostly sinusoidal with the main frequency component at the electrical rotational speed of the motor. The output of the power filter is optionally connected to the motor with a corrosion tolerant connector 15, e.g. implemented as a direct feedback connector, which enables a measurement of the voltage at the motor terminals without the influence of connector contact resistance. The motor actuator coils 3 are located inside the VAD and are magnetically coupled to the rotor magnets 4. The rotation of the rotor 4 induces a BEMF into the actuator coils 3 (i.e. motor coils). The position of the rotor 4 along an instable DOF modulates the shape of the BEMF and also induces its own BEMF into the actuator coils 3. The BEMF from rotor rotation and translation modifies the motor phase current and terminal voltage. Both can be measured by a BEMF replicator 12, which generates a digital or analogue copy of the induced BEMF. The BEMF replicator 12 can further be used to generate an electric or digital signal with the shape of the magnetic field within the actuator coils 3. The replicated waveforms are fed to a rotor position estimator 7 that estimates the linear and/or rotational rotor position. The method used by the rotor position estimator 7 can include an arithmetic formula that inverses the law of induction, a Kalman filter based on a motor model or a controller controlling a simulation model of the motor. The rotor position is used by the levitation controller 8 to control the motor driver 2 or 10 to adjust forces on the rotor to levitate the rotor within the VAD.

BEMF based rotor position sensing generates no signal when the rotor is stationary. Therefore, the rotor has to be spun before the levitation control can detach the rotor and levitate the rotor.

An additional or alternative measurement method to BEMF replication may be used. The following methods also enable detachment at standstill. In a first method, the high frequency impedance of the actuating coils 3 is influenced by the rotor position. The actuator coils have an inductance but also a certain amount of capacitance between the turns. This generates a RLC circuit with a resonance. The resonance frequency is usually located at several MHz and well above common PWM frequencies. The resonance frequency and quality factor can be influenced by the rotor position. Any high frequency magnetic field generated by the actuator coils 3 can generate an eddy current inside conductive parts of the rotor 4. The generated eddy current creates its own magnetic field that opposes the magnetic field generated by the actuator coils, modifying their impedance. This mechanism is commonly described as eddy current sensor and is state of the art, when used with a dedicated sensing coil. In certain aspects of this invention, however, the actuator coils are used as eddy current sensors.

In a second method, the impedance of the actuator coils 3 can, additionally or alternatively, be modified by coil impedance modulators 13. The coil impedance modulators 13 can be implemented as magnetically sensitive capacitors connected to the actuator coils, magnetically saturable components whose saturation due to the magnetic field generated by the rotor magnets change the reluctance of the magnetic flux circuit through the actuator coils or dedicated coils or an active electronic circuit using magnetic field sensors. An additional force is acting on the rotor, if magnetically saturable components are used inside the stator. Then, a compromise between high sensor signal and low rotor forces has to be made by using an optimal material, e.g. non oriented electrical steel, and amount of magnetically saturable material. A high frequency current has to be imprinted on the phase current to measure the phase impedance. All methods have in common, that they modulate the impedance of the actuator coils and therefore no dedicated wires, in addition to the motor wires, are used to read out their signal.

The impedance of the actuator coils is measured in the control unit with an impedance analyzer 14. The impedance analyzer excites the resonance or another high frequency inside the actuator coils and observes the phase current or terminal voltage to determine the impedance. This method is also limited by noise and benefits from a low noise motor driver and filtering of the switching noise. Alternatively, the motor driver switching action could excite the actuator coils with an RF current. The impedance signal is fed to another rotor position estimator 7 and also provides a rotor position signal to the levitation controller.

A third method of rotor position measurements (not shown) uses dedicated sensors like, but not limited to, hall effect, eddy current sensors, fluxgate sensors or ultrasonic sensors to measure the rotor position. The sensor signal is imprinted on the driveline signals. The signals could be frequency, amplitude or code modulated prior to imprinting them onto the driveline. A RF-receiver, similar to the impedance analyzer, detects the signals inside the control unit to provide a rotor position signal. In one embodiment, the actuator coils 3 are be used to oscillate the rotor in axial, radial, rotational or tilt direction at an ultrasonic frequency. Microphones or the actuator coils pick up the sound that originates from the rotor. The time delay or phase shift between acoustic sender and receiver can be used to measure the rotor position or volume flow through the VAD.

The levitation controller 8 uses one or multiple of the available rotor position signals to close the levitation control loop. Based on the motor and levitation operating the position measurements could be weighted or thrusted differently. To aid the weighting, the rotor position estimators 7 can optionally prove a signal quality indicator. If any of the signals deviate from another or a simulated pump model, an alarm or log entry can optionally be triggered.

The VAD system in Figure 10 can be implemented by using any combination of blocks from Figure 9 and Figure 10. It is e.g. possible to use BEMF or impedance based measurements with a switching mode motor driver or use only one of the provided methods for rotor position measurement.

The sensorless motor VAD as described in this application can also be combined with a dedicated bearing coil to either isolate the sensor and actuator signals from each other or to increase the efficiency of the levitation control.

Position Sensor Integration in Motor or Bearing Coil

Every levitation control loop needs a rotor position signal at its input. Commonly, dedicated sensors such as magnetic field sensors (Heart Mate 3, Ab-bott) or eddy current sensors (INCOR, Berlin Heart GmbH) are used to measure the rotor position. The sensor signal is either evaluated inside the pump with integrated pump electronics or transmitted to the control unit using additional wires in the driveline. Both options reduce the viability of the VAD system for use in children.

The presented sensor solution does not require dedicated sensors, complex electronic systems inside the pump or the addition of wires to the driveline. In most cases the motor structure is directly or indirectly used to measure the rotor position.

The presented concepts were developed for a disc shaped axial flux motor. However, the sensing methods can be used with other types of BLDC motors or even linear actuators e.g. voice coils.

Motor Model Based Rotor Position Sensing

When the distance between rotor-magnets and stator coils is increased, then less magnetic flux reaches the motor coils. This affects the characteristic of the motor setup. Characteristic motor parameters are e.g. the no-load-speed and stall torque. A weaker field or greater rotor-stator-distance will increase the no-load-speed and decrease the stall torque. During operation, neither of these parameters can be measured directly. However, both are defining the current, voltage and speed behaviour of the motor. A measurement of all three values can be used to calculate an estimation of the BEMF. The BEMF is related to the magnetic field through the motor coils via the law of induction:

(1)

$$V_{BEMF} = -N \frac{d\Phi}{dt} = -N \frac{d}{dt} \left( \int_A B \, dA \right)$$

with N - number of windings, $\Phi$ - magnetic flux through coils.
The field outside of a Halbach array rotor can be estimated to

(2)

$$B(x, z) = B_0 \, e^{ikx} \, e^{-kz}$$

with B_0 - magnetic field strength at the surface of the magnets, k - magnet wave number, z - distance from magnet surface, x - lateral position By inserting (2) in (1), we see that the BEMF is proportional to field strength and rotor speed:

(3)

$$V_{BEMF}(x, z = const) = -N \, c_{geo} \frac{d}{dt} \left( B_0 \, e^{ikx} \, e^{-kz} \right) = -N \, c_{geo} B_0 e^{-kz} \frac{d}{dt} \left( e^{ikx} \right)$$

with c_geo - coil geometry constant
The actually measureable BEMF is:

(4) = re(3)

$$re[V_{BEMF}(t, z = const)] = -N \, c_{geo} B_0 e^{-kz} \frac{d}{dt} cos(\omega t) = N \, c_{geo} B_0 e^{-kz} \omega \, sin(\omega t)$$

with $\omega$ - angular frequency, t - time
With known speed and BEMF value, the z position can be calculated. However, the BEMF cannot be measured directly while the motor is in operation under FOC. The only measureable motor voltage is the sum of BEMF, voltage drop over motor resistance and voltage drop over motor inductance (V_Mot, V_BEMF, V_R, V_L in Figure 11). Using a measurement of the motor current I_Mot, the voltages inside the motor V_L and V_R can be calculated. After measuring the voltage over the motor terminals V_Mot, an estimation of V_BEMF can be calculated.
The motor speed can be estimated e.g. with a frequency analysis of the BEMF. With these measurements, the z-axis rotor position can be estimated:

(5)

$$e^{-kz} = c_{zest} \frac{V_{Mot} - R I_{Mot} - L \frac{d I_{Mot}}{dt}}{\omega}$$

with c_zest - z position estimation constant.
Every phase of a three phase BLDC motor can be modeled like Figure 11. It can be sufficient to measure only one phase to gain a z-position measurement. However, by measuring all three phases a more accurate measurement can be performed.

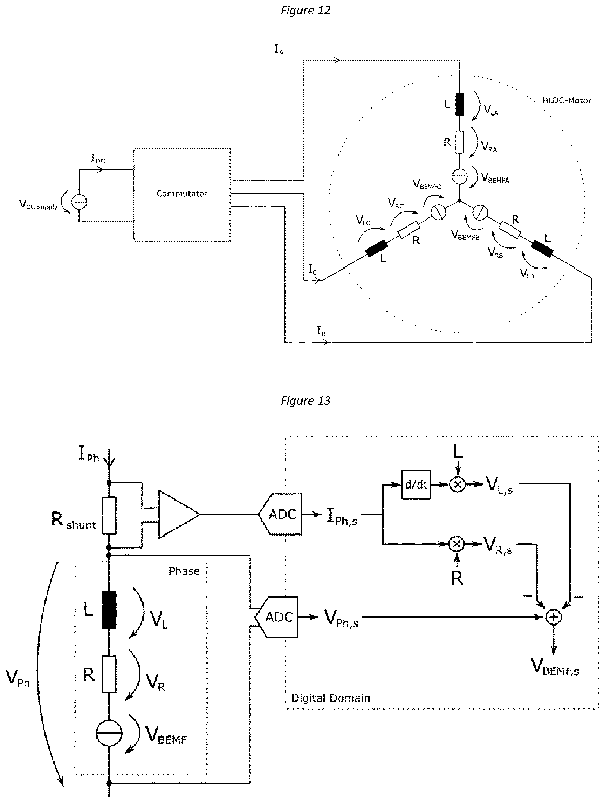

An accurate measurement of the DC-equivalent motor-current of a BLDC motor can be taken by measuring the current in the supply of the commutator circuit (see, for example, Figure 12).

These methods are accurate enough to commutate the motor using the estimated BEMF and also accurate enough to get an averaged z-position measurement. However, if fast and accurate measurements of the BEMF are necessary, e.g. for a position control loop in a levitation device, then improvements may be necessary to increase the signal-to-noise ratio.

The presented improvements for measuring the BEMF are reducing the systematic noise of the current measurement, voltage measurement, the subsequent filtering and the position estimation. Especially the high frequency noise reduces the accuracy of the inductor voltage estimation. This is due to the high-pass characteristic of the differentiation operation in the inductor voltage estimation.

Direct VL Sampling with an Inductive Shunt

A circuit for calculating the BEMF according to the prior art is shown in Fig. 13. Embodiments to overcome the limitations of the circuit of Fig. 13 will be explained below.

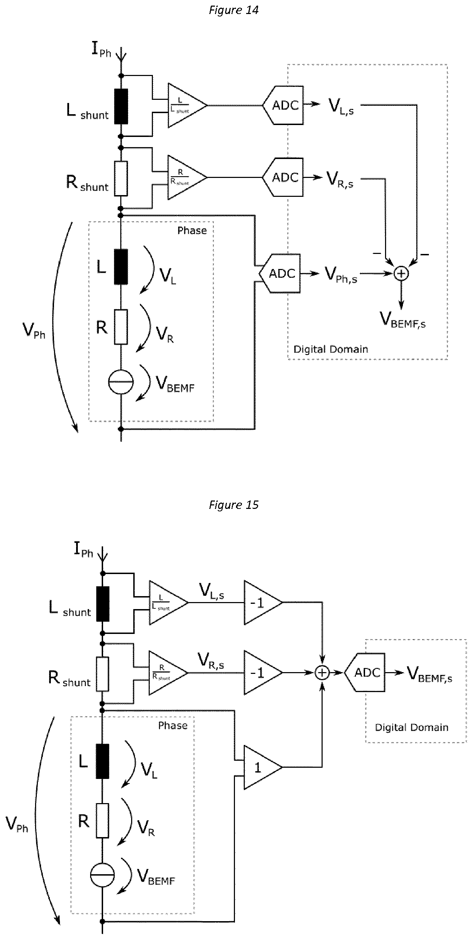

An inductive shunt is connected in series to the resistive shunt. The motor current and its transients also flow through the resistive and inductive shunt. The voltage over the inductive shunt is a scaled image of voltage over the motor inductivity. An analog voltage sample of VL can be generated by amplifying (rescaling) the voltage over the inductive shunt. The calculation of VBEMF can be realized in the digital domain (Figure 14) or analog domain (Figure 15).

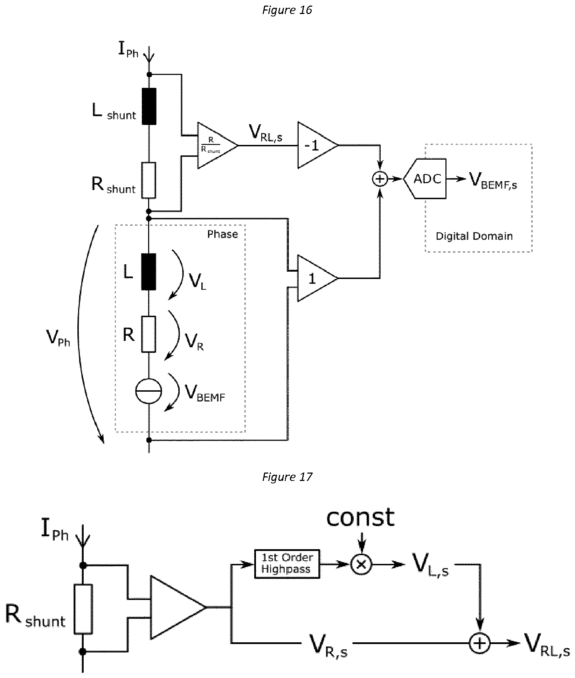

If the ratio between resistive shunt and inductive shunt is equal to the ratio between motor resistance and motor inductance, then only a single combined resistive-inductive shunt can be used (Figure 16).

The inductive sampling could also be emulated with analog differentiation using a high-pass filter (Figure 17).

Direct BEMF Integration

The estimations in chapter "Motor Model Based Rotor Position Sensing" are only valid for slow movements in the z-direction. A magnetically destabilized z-axis can reach speeds where the change in magnetic field also induces a significant voltage in the motor windings.

This leads to wrong rotor angle and z-distance estimations when conventional BEMF vectors are evaluated.

To detect fast movements in the z-direction, it is advantageous to know the actual magnetic field strength inside the phase windings.

Due to the relation between BEMF and magnetic flux:

$$(6) \qquad V_{BEMF} = -N\frac{d\Phi}{dt}$$

the flux can be calculated from the BEMF:

$$(7) \qquad \Phi = -\int_t \frac{V_{BEMF}}{N} dt = BA$$

with $\Phi$ - flux, N - windings, A - coil area, B - coil field

The integration creates an offset due to the unknown integration constant. Therefore, only the high pass characteristic of the flux can be determined with high accuracy. However, the low pass characteristic of the z-axis movement can be extracted directly from the BEMF measurements.

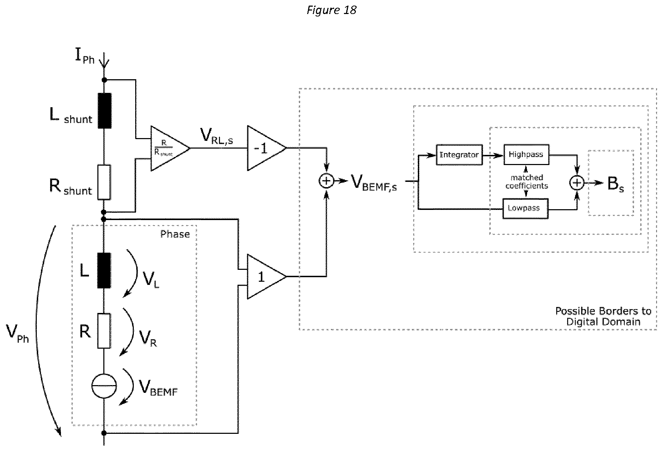

Both measurements can be combined using a matched high-pass-low-pass filter to obtain a fast and accurate rotor position measurement. The integration and matched filtering can be accomplished both in the analog and digital domain. Figure 18 shows the flow chart and possible transitions from analog to digital domain.

The rotor position can be directly calculated from the magnetic field strength using formula (8):

(8)

$$B(x, z) = B_0 \, e^{ikx} \, e^{-kz}$$

Motor Winding Eddy Current Sensors

Eddy current sensors detect the presence of a conductive target in the field of a high frequency coil. Normally, motor coils are not suitable for eddy current measurements, because they would primarily detect the presence of the iron core. In a further aspect of the invention, it has been found that Ironless (stator-core- and yoke-less) motors do not have this problem, but still have enough efficiency to drive the pump. In this case, the windings of ironless motors can be used to take eddy current readings. The target could be a copper plate in the rotor, the rotor titanium housing or conductive magnet material.

In operation, a high frequency current is imprinted on the actuator coil current. The motor driver and eddy current sensing circuit can be isolated from each other using passive filters. A big gap between eddy current frequency and motor operation frequency or PWM frequency benefits the filter design.

Active filters, e.g. lock-in amplifier, could also be used for the extraction of the eddy current signal. One of the biggest noise sources are the harmonic components of the PWM signal. Motor drivers with no or reduced switching noise are especially suitable for simultaneous eddy current measurements. Such drivers are explained further below.

Eddy Current Rotor Angle Measurement

BEMF sensing cannot be used at very low speeds. This means, that the axial rotor position cannot be measured with BEMF below a certain speed. A maglev pump that uses only BEMF to measure rotor angle and axial position would need to spin the rotor before liftoff. Sufficiently good backup bearings are necessary to allow this type of operation.

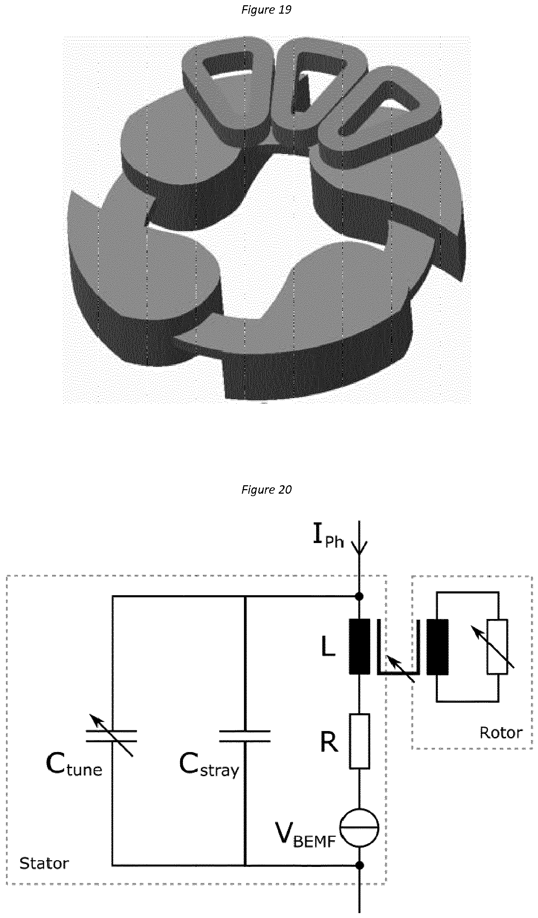

Eddy current sensors do not rely on a rotation of the rotor and are generating a signal even at 0 rpm. The rotational rotor position could be measured by comparing multiple eddy current sensors in multiple motor phases (Figure 19). The rotation detection could even be implemented in a shrouded rotor. Dedicated copper targets could be placed on top of some of the magnets, making them better targets. An alternative or addition to this is to reduce the conductivity of some magnets by segmenting and isolating the magnets. This method is used excessively in high performance BLDC motors to reduce eddy current losses.

To improve the signal to noise ratio, the BLDC commutation could be operated in six step mode, where one of the phases carries no current. This phase is then used for the eddy current measurement by imprinting an RF current.

Field Dependent Resonant Circuit Tuning

At high frequency, the stray capacitance of the motor windings creates a resonator with the winding inductance. Most commonly, the eddy current sensors are operated close the self-resonant frequency of the sensing coil. The eddy current modifies the inductance value, so that the resonant frequency changes (Figure 20, right panel: Rotor). Another option to modify the resonant frequency is to tune the stray capacitance. Because of the high frequency, only very small capacitances are necessary to shift the resonant frequency significantly with an additional tuning capacitor (Figure 20, left panel: Stator).

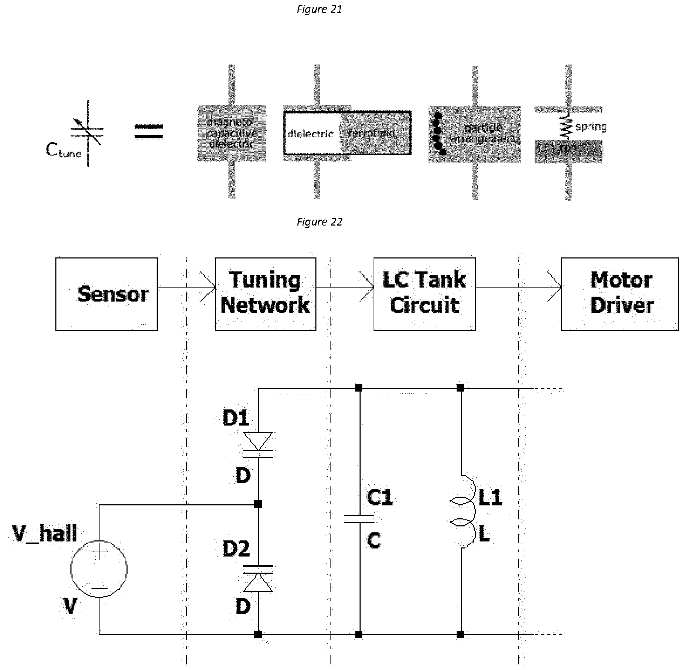

The tune capacitor would be built so that the capacitance changes depending on rotor position. Either magnetic field strength or BEMF could be measured. Possible implementations include capacitors filled with a magneto-capacitive dielectric (Figure 21, left).

The tuning capacitor could alternatively be partially filled with ferrofluid to change its dielectric constant when the ferrofluid is moving under the contacts (Figure 21, left).

Instead of changing the dielectric, the geometry of the tune capacitor could be modified by the magnetic field due to reluctance forces (Figure 21, right).

With more space available, any electronic sensor can be used to tune a varactor diode with its output signal. The capacitance of the varactor diode could then tune the resonant frequency (Figure 22). Possible sensors include magnetic field sensors, capacitive distance sensors, RF transceivers, ultrasonic transceivers or dedicated BEMF sensing coils as well as flow or pressure sensors. An active sensor circuit could be supplied with parasitic power from the motor-lines, requiring no extra driveline leads.

Low Noise Motor Driver

The purpose of a motor driver is to generate a desired amount of torque in a motor. The torque is depends only on the rotor position and the currents in the phase coils. The most common method to control the current is pulse width modulation (PWM). PWM switches the phase voltage rapidly between multiple voltage levels. Due to motor inductance, the current cannot follow the fast changes in applied voltage and creates a triangular current waveform. The exact amount of current is controlled with the PWM duty cycle. The current waveform is often smoothed using additional inductivities in series to the motor.

The major advantage of PWM is its energy efficiency. While one voltage level is applied to the phases, only very small resistive losses are created in the switching elements. Some additional losses are produced in the transition from low to high and high to low phase voltages. These losses can be reduced by keeping the transition time as short as possible.

Small transition times are creating high frequency components. These frequency components can be an issue for electromagnetic emissions (EMI) and attached sensor devices. Common measurements against high emissions are passive filters which either conduct the high frequency current components to ground or burn them as resistive losses. Small valued capacitors in combination with common mode chokes are usually sufficient to make a motor driver EMI compatible.

The high frequency components do not only radiate out of the driveline, they are also conducted to the sensor electronics attached to the motor-driver. The most common form of sensor in motor drivers are phase current sensors. They are used to generate a current control feedback loop to accurately control the phase current. In sensorless BLDC motor drivers these current sensors are also used to estimate the rotor angle. Common rotor angle estimation methods include zero crossover detection, BEMF estimation or a model based estimator approach.

To reduce the influence of fast switching times on the current measurement, it is quite common to synchronize the current sampling to the PWM switching, so that the time between sampling and switching is maximal or constant. It is also state of the art to use low pass filtering on the sensor signal to suppress switching noise.

However, state of the art motor driver and current sensor concepts are neither sufficient for simultaneous motor operation and eddy current measurement as presented in chapters "Motor winding eddy current sensors", "Eddy current rotor angle measurement" and "Field dependent resonant tuning circuit" nor are they providing a high signal to noise current or voltage measurement that could be used to levitate a pediatric VAD rotor.

The BEMF can be estimated much more accurate in the absence of switching noise. BEMF detection which is normally just sufficient for motor operation above a certain minimal motor speed, can probably also be used for fast rotor position measurement (see chapters "Motor model based rotor position sensing", Direct $V\_L$ sampling with an inductive shunt" and "Direct BEMF integration").

Passive RF and PWM Filters

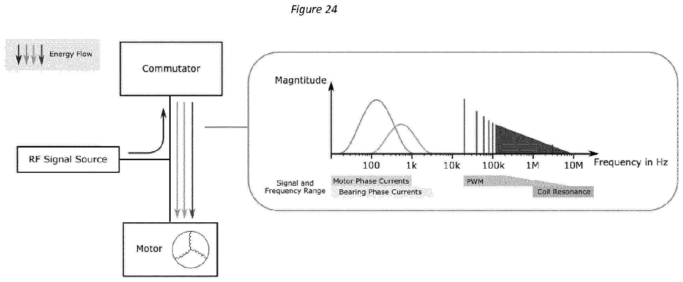

A high frequency current must be imprinted in the motor coils to perform eddy current measurements with the motor coils according to sections "Eddy current rotor angle measurement" or "Field dependent resonant tuning circuit". When connecting a high frequency source to the driveline (see Figure 24), the RF energy will flow into the motor driver. This is due to the high impedance of the motor at motor coil resonance and a relatively low input impedance of the motor driver at the motor coil resonance frequency of several MHz.

The load on the RF source is high but no RF current flows in the motor coils. Because the motor coil resonance is not excited, the RF current has no dependency on the specific impedance characteristic of the motor coils. Therefore, no rotor position sensing is possible.

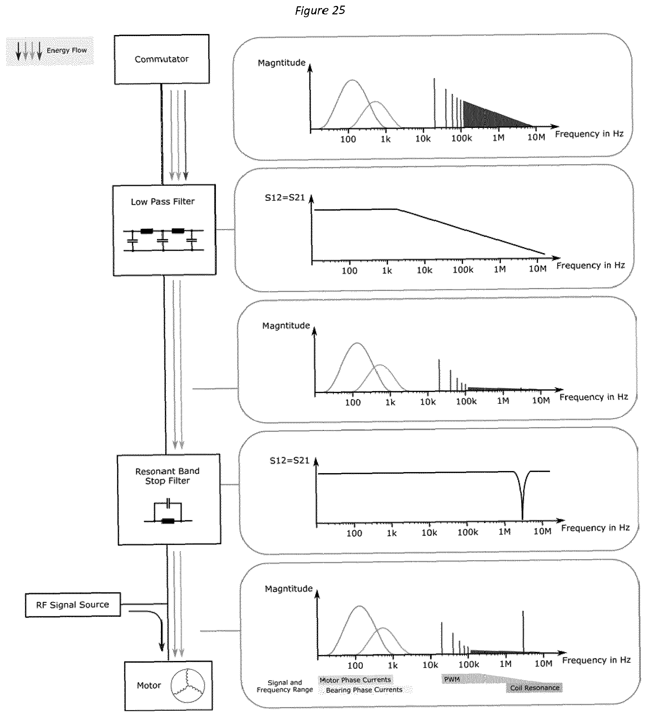

To direct the RF current to the motor, the RF input impedance of the motor driver must be high at least at the resonance frequency of the motor coils. Resonant band stop filters and/or low-pass filters can be used to increase the impedance at a specific frequency (see Figure 25). With this method a high filter quality can be achieved with only two passive components per phase.

Now the RF current can excite the motor coil resonance. To measure the impedance accurately, no other RF source should excite the resonance. However, the harmonic components of the PWM can reach up to several MHz. This can significantly reduce the signal to noise ratio of a sensitive rotor position measurement system.

To prevent the PWM voltage harmonics from being converted into harmonic motor currents, a low pass filter can be used (see Figure 25). A wide range of frequencies (10Hz - 2kHz) has to pass this filter while a broad spectrum of PWM harmonics need to be suppressed. Passive low-pass filters (with multiple stages) can at least reduce the harmonic PWM components.

More filter elements may be necessary to couple the RF source to the driveline or to prevent the voltages on the driveline from damaging the RF source.

Current and Voltage Sensor Filtering Options

The common approach to suppress PWM noise on shunt current sensors is to synchronize the sampling to the PWM frequency.

The shunt signal is often also filtered using passive low pass filters. These filters can be made more effective than the filters in series to the motor phases, because only a low sensing current instead of the motor phase current is flowing in the filter.

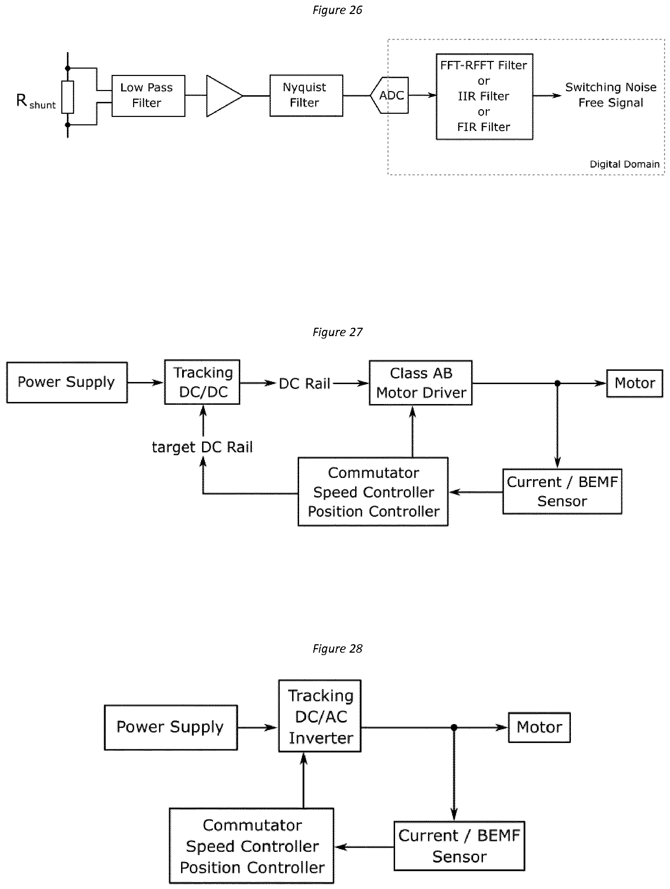

A higher isolation can be achieved with digital filtering (see Figure 26). The frequency components of the signals to be suppressed must be within the nyquist frequency range. Therefore, the sample rate must be significantly higher than the PWM frequency.

Class AB Motor Driver with Bus Voltage Control

The switching noise of a motor driver can be significantly reduced by not switching the output transistors at all. Instead, the output stage uses a class AB topology to linearly control the current in the motor phases. The drawback of a class AB output stage is its limited efficiency of maximum 78% at full output voltage swing. The efficiency is much lower if the maximum output voltage of the motor driver is much lower than the DC-rail voltage.

To keep the efficiency of the motor driver at an acceptable level, the DC rail voltage can be controlled with a DCDC converter to be just above the maximum voltage swing (see Figure 27).

Tracking DC/AC Motor Driver

Multiple Tracking DC/DC converters can be combined into an AC inverter (see Figure 28). The AC inverter can directly control a motor phase current. The difference between a push-pull motor driver and an AC inverter or tracking dc controller is that the voltage waveform at the output of an AC inverter can be much smoother than the output from a PWM motor driver.

Impedance Measurement Between PWM Harmonics

The harmonic components of a PWM are placed at $n*f\_PWM$, with n {1,2,3,4,...}. That means that the switching noise is confined within certain areas of the spectrum. The noise level in between these areas can be much lower.

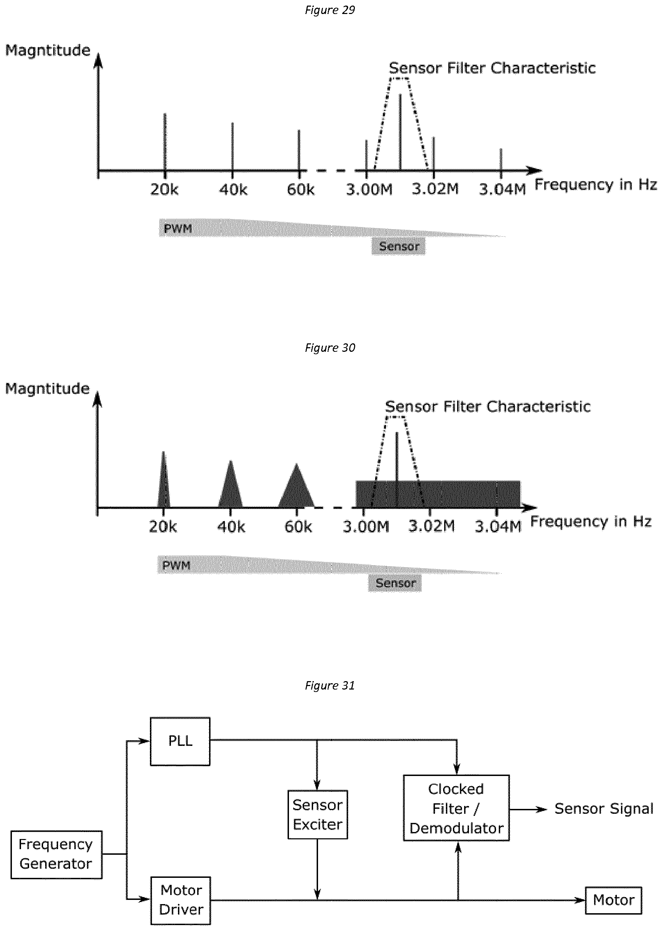

A narrow band RF sensor signal could be placed in such a low noise frequency range. However, the input filter of the sensor needs to be capable of suppressing the neighbouring PWM peaks (see Figure 29). This frequency placement increases the requirements for the frequency stability PWM and sensor signal. Figure 30 illustrates the origin of these requirements. If the PWM jitter at the fundamental PWM frequency (PWM period) is a certain amount, then the jitter at the n-th harmonics is n times as wide. At high frequencies the PWM spectrum merges into a continuous band without gaps. This is because the distance between harmonics is constant but the jitter gets wider with higher frequencies, overlapping at some point. At this point it is not possible to extract the sensor signal without also picking up the switching noise.

Jitter is a mathematical method to describe frequency variations. Even with jitter, at any instant in time the PWM spectrum consists of single frequency peaks and looks like the spectrum in Figure 29. Only after averaging over time the spectrum looks like displayed in Figure 30. Because of this, the sensor frequency can always be placed in between the PWM harmonics. However, the sensor frequency and filter characteristic have to be adjustable.

The PWM frequency, sensor frequency and switching filter could be synchronized to the same clock source. This can keep the sensor frequency always between the PWM harmonics. The sensor filter could be realized using a lock-in filter, which can easily be tuned with a clock source. Phase locked loops (PLL) and frequency dividers can be used to keep the PWM and sensor frequency at a certain ratio (see Figure 31). Instead of the sensor frequency being clocked by a PLL, the motor driver could be synchronized to the sensor frequency. The important characteristic is that both are clocked directly or indirectly by the same frequency generator, so they see the same jitter.

PWM Harmonic Driven Eddy Current Sensors

Instead of isolating the eddy current sensor signal from the PWM harmonics, it is also possible to use one of the PWM harmonic components to excite the motor coil resonance. Here, a high frequency PWM with short switching times can actually be advantageous. High frequency PWM motor drivers are also optimal for ironless BLDC motors. The low inductance of iron-less motors can make additional inductances in the motor driver necessary. These inductances are smaller or can be omitted completely in high frequency motor drivers.

The resonance signal could be extracted from the motor signal using lock-in filters which are clocked directly or indirectly by the same clock source that also clocks the PWM cycle.

Highly integrated motor drivers ICs achieve high switching speeds in small packages, enabling small control units.

Resonant Decay PWM Excited Eddy Current Sensors

The resonance of eddy current sensors can be characterized by the resonant frequency and the resonant quality factor. Most often the frequency is evaluated. However, in some applications the quality of the resonance can be more sensitive. The switching instant of a PWM cycle can often be approximated as a dirac impulse. This dirac impulse excites the resonant frequency of the eddy current sensor. The resonance then decays until the next switching instant. The quality factor is directly related to the decay time.

To amplify the excitation, the PWM switching could include a burst of transitions instead of a single transition. This could also shape the harmonics spectrum to concentrate the energy of the PWM harmonics to be close to the eddy current resonance.

Direct Feedback Connectors

For an accurate measurement of the BEMF according to chapter 3.3.1, 3.3.2 or 3.3.3 it can be advantageous to know the motor phase voltage accurately. Between the motor and the control unit, where the voltage can be measured, there are the driveline and the connector. Especially the connector suffers from gradual and sudden resistance variations. Movement of the contacts suddenly changes the contact point and contact pressure and therefore the contact resistance. The contacts within the connector are also much more exposed to the environment than the wires within the driveline. This can lead to corrosion which gradually changes the contact resistance and also increases the sudden resistance variations due to a non-uniform corrosion.

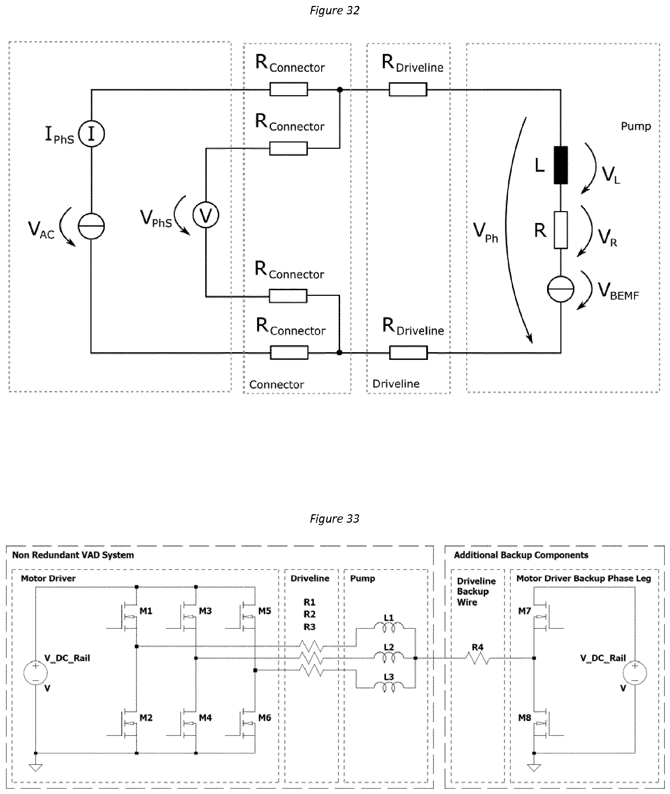

The proposed solution uses a connector with additional contacts to measure the phase voltage without the influence of the connector resistance (see Figure 32). The amount of wires in the driveline is not affected by the direct feedback connector.

Power Over Motor Lines, Power Line Communication and Motor Line Communication

Power over motor lines, power line communication and motor line communication are methods to read out and supply sensors inside a VAD without adding additional wires to the driveline. The methods disclosed in the patent application WO2018206754A1 can be used to transmit the signal of dedicated sensor signals from an implanted VAD to the controller. Possible sensors include, but are not limited to, rotor position sensors, acceleration sensors, gyroscopic sensors, blood flow sensors and blood pressure sensors.

Fail-Safe Motor, Bearing, Driveline and Motor Driver

Driveline defects and driveline infections make up a significant portion of VAD therapy failures. The common approach to reduce driveline defects is to use backup wires inside the driveline. If any wire breaks, then the corresponding backup wire can take over. An alarm usually informs the user about the state of emergency.

The common approach doubles the number of wires inside the driveline. A motor-only VAD like the HVAD has 6 instead of 3 wires in its driveline due to redundancy. This however increases the cross section of the driveline and therefore increases the risk of driveline infections.

Other VAD systems line Heart Mate 3 are reducing the number of driveline wires by supplying the pump with dc current and placing the motor driver or bearing driver inside the pump. With this approach only one or two extra wires are necessary for a fail safe driveline. The additional electronic components inside the pump increase its size and reduce the possible patient population.

The proposed fail-safe approach for external motor driver VAD systems has been published for different applications. However, no currently available VAD system uses this technology.

The proposed fail safe method utilizes a three phase BLDC motor. The driveline contains three wires which connect the motor phases to a phase leg each. The motor coils have to be connected in star (instead of delta) configuration. The starpoint is normally connected inside the motor and is not accessible from the outside. An additional backup-wire in the driveline connects the starpoint to an additional backup phase leg inside the drive line (see Figure 33).

The requirement for motor, bearing or sensor operation of a BLDC motor is that at least two of the motor windings must be supplied with an independent current. If for example in the non-redundant VAD system in Figure 33 the coil L3 or the wire R3 or one of the switches M5 or M6 fails with an open connection, then the current in L3 is zero and cannot be set from the outside. The remaining coils L1 and L2 are now connected in series and are therefore carrying the same amount of current. Only an oscillating, instead of a rotating, magnetic field can be created with this configuration and motor operation is usually not possible.

By utilizing the additional driveline wire R4 and the additional phase leg M7 and M8, the current of L1 and L2 is again independent of each other. This allows creating a rotating magnetic field and operates the motor in a normal two phase fashion.

If a dedicated bearing coil is necessary, the bearing coil can be connected between the BLDC star point and a fourth driveline wire. This configuration can keep the motor in operation if any of the wires in the driveline, a motor coil or a phase leg fails with an open connection. The mayor drawback is that in the event of a failure the bearing can no longer be operated independently of the motor. This may be an option if the VAD has sophisticated backup bearings.

A major drawback of the presented configuration is that all the bearing current passes through the motor wires, creating additional losses and magnetic fields. With zero force control the amount of bearing current should be small compared to the motor current, reducing the impact of the drawback.

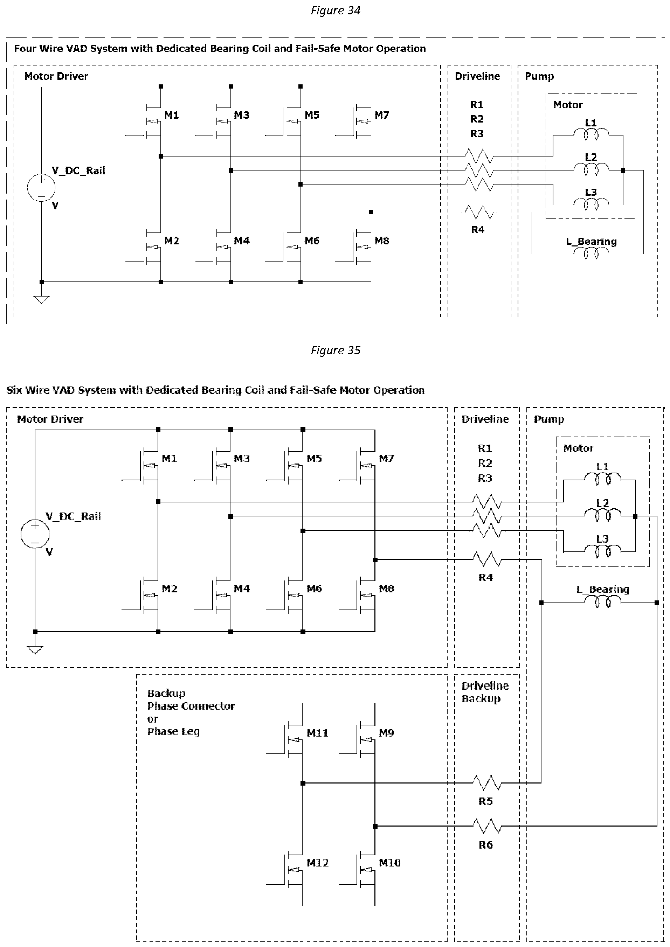

With six wires the bearing and motor operation can be continued after an open connection failure in the driveline (see Figure 35).

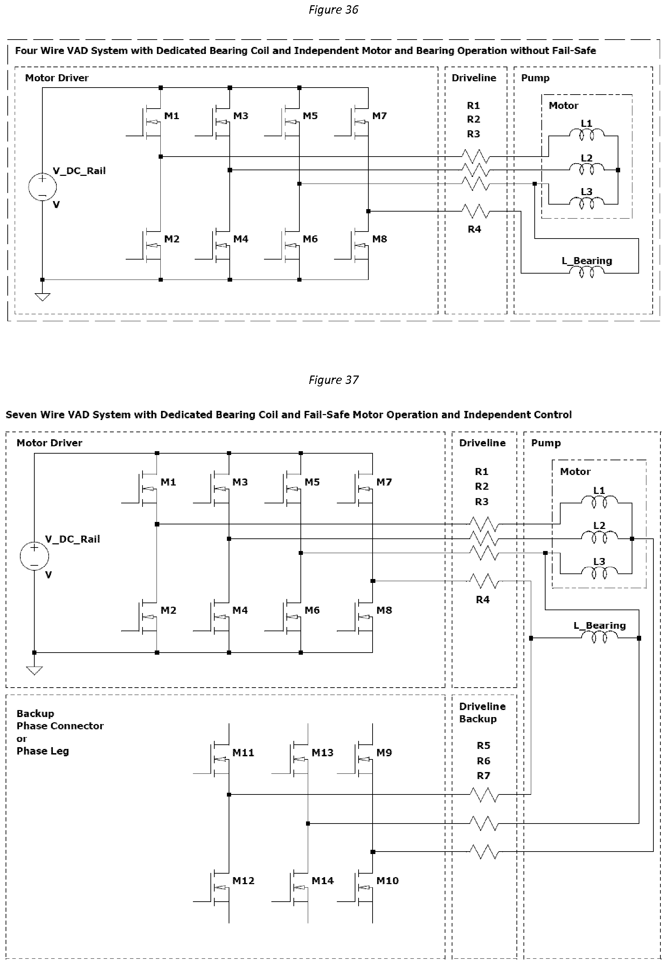

The four wire BLDC motor with dedicated active bearing configuration (Figure 36) can be used when the risk to the patient due to a large driveline or an inefficient bearing is greater than the risk of an open connection failure.

The seven wire BLDC motor with dedicated bearing structure (Figure 37) makes sure that even with an open connection failure the motor can be operated and the active bearing does not lose any efficiency.

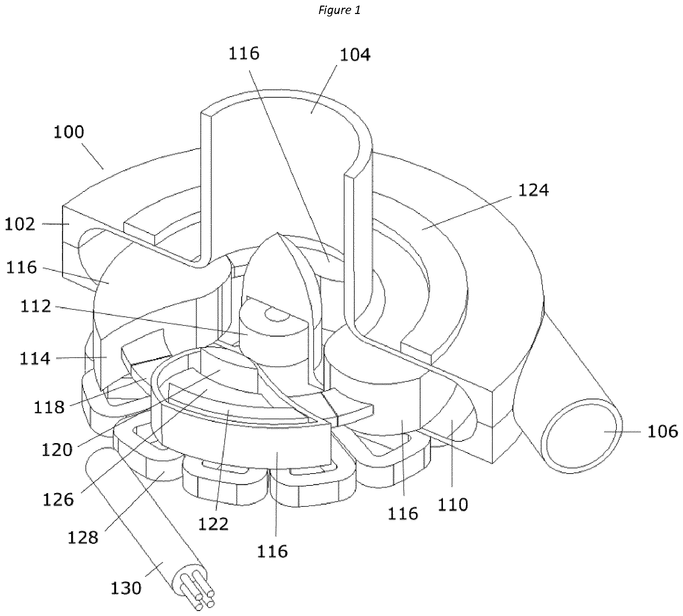

Figure 1 shows an exemplary blood pump. The blood pump 100 includes a housing 102 with an axial inlet 104, and an outlet chamber 106. The outlet chamber 106 includes an outlet 108, which can be connected to a graft or tube to be connected to the vascular system such as the artery. The inlet 104 can be inserted into the apex of a heart ventricle or can also be attached to a graft or tube which is attached to the vascular system.

The outlet chamber 106, which may be designed as a volute, includes a back plate 110 away from the inlet. In the example shown, the back plate 110 includes a central spire which extends in the direction of the axial inlet and houses a permanent magnet 112. The chamber 106 houses a magnetically levitatable impeller 114 including four blades 116 (some are shown in a cut-through view to show the inside of the blade) which are connected to each other via webs 118. The impeller includes a plurality of permanent magnets: Each blade includes a permanent magnet 120 which acts as the counterpart to permanent magnet 112. This system of magnets is part of a passive radial magnetic bearing. The impeller further includes an optional tilt bearing magnet 122 (preferably also in each blade), which interacts with a magnetic ring 124 placed on the housing, thereby forming a tilt bearing.

Furthermore, each blade includes a rotor magnet 126, which interacts with the motor coils 128. The motor coils are placed on the far side of the back plate and are ironless, preferably copper windings. The power, i.e. current and voltage, within the motor coils is controlled via a control unit (not shown in Figure 1) which is preferably placed outside of the human body. The coils and the control unit are connected via a drive line 130. The drive line 130 includes four wires, wherein one of the wires can be used as a redundant wire. The wires are used to control the motor coils, such that the impeller can be rotated and the position of the rotor, at least in the axial direction, can be measured via one of the control schemes, methods or circuitry outlined in this application. Since the blood pump has no separate rotor position sensor, the wires needed within the drive line can be reduced compared to conventional blood pumps.

In other embodiments the pump may have further sensors; however, the motor coils are used for sensing and measuring a position of the rotor.

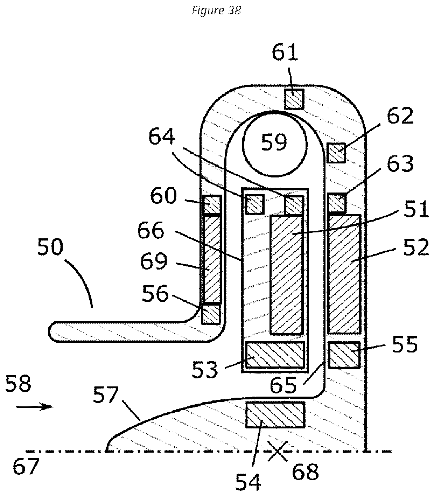

Figure 38 shows a blood pump that can take advantage of the presented improved bearing concept. The blood pump 50 contains an inflow 58 and at least one outflow 59. A rotor 66 is driven by actuators comprising (or in some embodiments consisting of) rotor-magnets 51 and actuator-coils 52 and optional additional or alternative actuator-coils (motor-coils) 69. In operation, the rotor rotates mainly around the pump axis 67. The rotor 66 is magnetically levitated inside the bloodpump 50. A passive magnetic bearing comprising at least two of the magnetic components 53, 54, 55 or 56 limits the radial movement of the rotor. The radial bearing is instable in axial direction and can have an instable equilibrium position. The actuator components 51, 52 or 69 can be used to control the axial rotor position to the instable equilibrium position or another predefined axial rotor position. Tilting of the rotor around the tilting point 68 is limited by one or more passive magnetic or bearings comprising at least two of the magnetic components 60, 61, 62, 63 or 64. The tilting could alternatively or in addition be controlled using the actuators components 51, 52 or 69. The blood pump can contain a central hub or spire 57 to hold the radial bearing component 54 in place. The hub can be minimized or omitted, if the radial bearing does not rely on the magnetic component 54. The transition from the hub 57 to the back plate 65 can be implemented gradually to improve fluid flow and hemodynamics. The actuator coils 52 or 69 can be positioned near the outflow, near the inflow or at both locations.

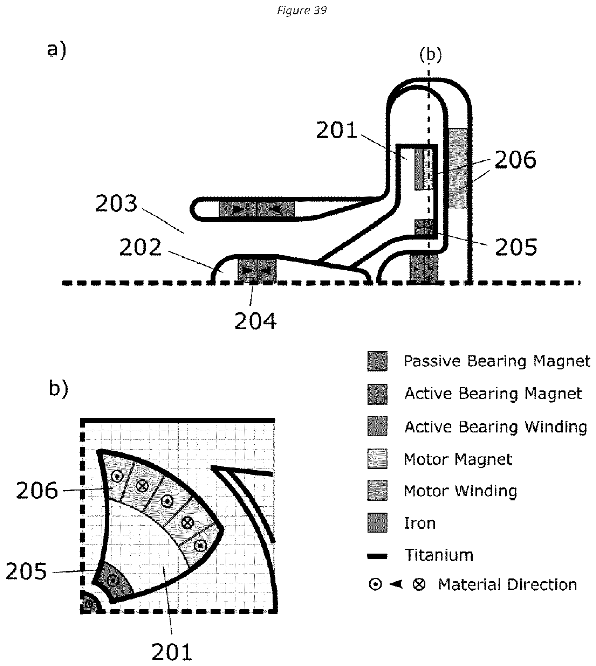

Figure 39 shows another embodiment of a blood pump in a cross-section view (Figure 39 a) and a top-view of a section of the rotor (Figure 39 b). The rotor blades 201 are connected by a central rotating hub 202 that can extend into the inflow 203. The rotating hub contains a radial bearing 204 and the rotor blades are containing an additional passive magnetic radial bearing 205. Both passive magnetic bearing are stabilizing the rotor in radial direction and tilt direction. The instable axial position is controlled by the actuator 206.

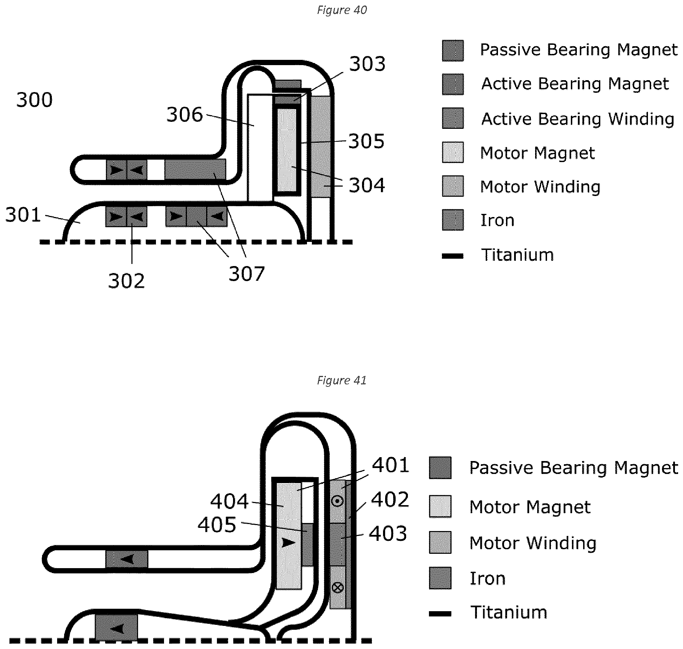

Figure 40 shows a blood pump 300 with a central hub 301 and two axially separated passive magnetic bearings 302 and 303. The magnet components of a motor or actuator 304 are placed inside a disc 305 inside a volute near the outflow or inside the rotor blades 306. The magnet disc is connected to the central hub via pump blades 306. An active magnetic actuator 307 could also be placed in the inflow and central hub. To increase the efficiency of the pump, motor 304 and dedicated active magnetic bearing 307 could be used together to control the axial rotor position, and/or rotor speed and/or tilting motion.

Figure 41 shows a variation of the blood pump 300 shown in Figure 40, where the radial magnetic bearing 302 inside the volute is omitted. The bearing function is now carried out by the motor 401, which contains a magnetically conductive back plate 402 or teeth 403 which are attracted by the rotor magnets 404. A magnetically conductive field concentrator 405 could be used to optimize the bearing capabilities of the motor bearing.

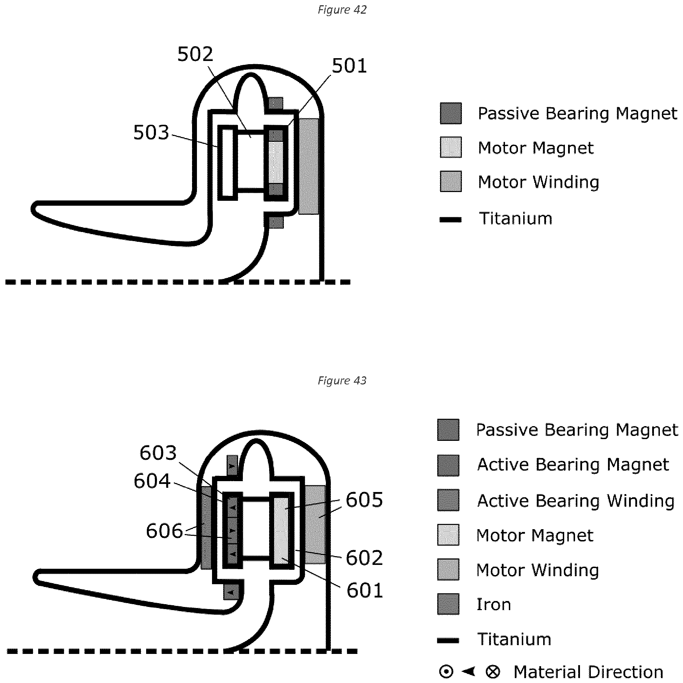

Figure 42 shows a variation of the blood pump in figure 1 or 38. The magnetic components of the rotor are contained in a disc 501. The pump blades 502 are located on top of the rotor disc 501. A second disc 503 could optionally be placed opposite to the first disc. The magnetic components could be located in either or both discs.

The blood pump in figure 43 shows one variation of the blood pump displayed in figure 42. The motor magnets 601 are located in one disc 602 and the bearing magnets 603 are located in the other disc 604. The field of the bearing magnets 603 can be used for an active axial bearing. Motor 605 and active axial magnetic bearing 606 could share the load of the rotor axial position control to increase maximum force and/or efficiency.

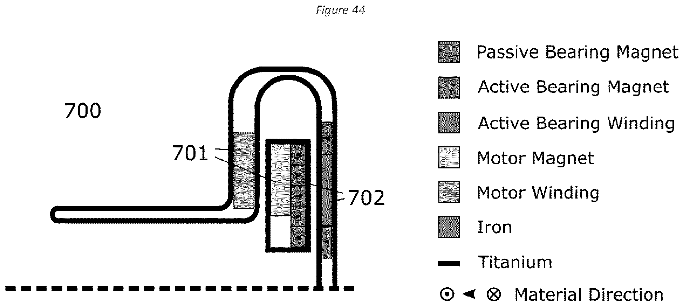

Figure 44 shows the load sharing of motor 701 and active axial bearing 702 implemented in a shroudless rotor centrifugal pump 700. Motor 701 and active bearing 702 can be separated axially as shown or radially.

**Claims**

1. Blood pump system, in particular a ventricular assist device, VAD, system including

    - a blood pump, the pump comprising:

        - a housing, including an inlet and an outlet, preferably an axial influx and a tangential outflow;
        - a motor actuator, wherein the motor includes a plurality of motor coils (for driving an impeller);
        - an impeller, wherein the impeller is located in the housing and includes a plurality of rotor magnets;

    the system further comprising:

        - a drive line;
        - a control unit for controlling operation of the pump, the control unit configured to:

            - operate the motor, such that the impeller rotates around an axis; and
            - measure the rotor position in a direction along the axis using at least one of the plurality of the motor coils.

2. Blood pump system according to any of the previous claims, wherein the control unit is configured to reduce or eliminate switching noise from a motor driver.

3. Blood pump system according to any of claims 1 or 2, wherein an output stage of the motor driver includes filter elements for filtering out high frequency signals.

4. Blood pump system according to claim 3, wherein a high frequency signal is added to the filtered motor driver output.

5. Blood pump system according to any of the previous claims, wherein measurement of the motor currents includes a measurement of the motor coil impedance, preferably the high frequency motor coil impedance.

6. Blood pump system according to any of the previous claims, wherein a motor internal back-electromotive force is replicated outside the motor, preferably using inductive shunt voltage measurement.

7. Blood pump system according to any of the previous claims, wherein a magnetic field strength is replicated in an electrical or digital signal outside the motor, preferably using a back-electromotive force replica and a matched pair of high-pass and low pass filter elements.

8. Blood pump system according to any prior claim, wherein the control unit is configured to reduce voltage transients in the driveline or is configured to reduce trapezoidal or triangular current wave-forms with respect to the sinusoidal current waveforms.

9. Blood pump system according to the previous claim, wherein the control unit includes a dc-dc converter, or includes class AB amplifiers, and/or passive filter elements.

10. Blood pump system according to any of the previous claims, wherein the driveline includes no more than four wires, preferably three wires and one redundant wire.

11. Blood pump system according to any of the previous claims, wherein the blood pump includes a passive magnetic radial bearing and/or a passive magnetic tilt bearing.

12. Blood pump system according to any of the previous claims, wherein the blood pump includes an active axial magnetic bearing.

13. Blood pump system according to any of the previous claims, wherein the motor is an axial flux motor, preferably an ironless axial flux motor.

Figure 1

EP 3 827 852 A1

*Figure 2*

*Figure 3*

Figure 4

Figure 5

Figure 6

Figure 7

Figure 8

ACTUAL GAP x                                                    ESTIMATED GAP x̃

CONTROLLER

+    −

ENVELOPE
FILTER

ENVELOPE
FILTER

ACTUAL
ELECTROMAGNET

i                    ĩ

SIMULATED
ELECTROMAGNET

VOLTAGE ACROSS COIL

Figure 9

1              2                                         3            4
Power       Switching                               Actuator      Rotor
Source        Mode                                    Coils
            Motordriver

Levitation    Rotor                      Filter    Waveform
Controller   Position                              Sampling
             Estimator
    8            7                          6           5

Figure 10

Figure 11

*Figure 12*

*Figure 13*

Figure 14

Figure 15

Figure 16

Figure 17

Figure 18

Figure 19

Figure 20

Figure 21

Figure 22

Figure 23

Figure 24

Figure 25

*Figure 26*

R_shunt — Low Pass Filter — [amplifier] — Nyquist Filter — ADC — FFT-RFFT Filter or IIR Filter or FIR Filter — Switching Noise Free Signal

Digital Domain

*Figure 27*

Power Supply → Tracking DC/DC → DC Rail → Class AB Motor Driver → Motor

target DC Rail

Commutator Speed Controller Position Controller

Current / BEMF Sensor

*Figure 28*

Power Supply → Tracking DC/AC Inverter → Motor

Commutator Speed Controller Position Controller

Current / BEMF Sensor

Figure 29

Magntitude

Sensor Filter Characteristic

20k  40k  60k  3.00M  3.02M  3.04M  Frequency in Hz

PWM

Sensor

Figure 30

Magntitude

Sensor Filter Characteristic

20k  40k  60k  3.00M  3.02M  3.04M  Frequency in Hz

PWM

Sensor

Figure 31

PLL

Sensor
Exciter

Clocked
Filter /
Demodulator → Sensor Signal

Frequency
Generator

Motor
Driver

Motor

*Figure 32*

*Figure 33*

*Figure 34*

**Four Wire VAD System with Dedicated Bearing Coil and Fail-Safe Motor Operation**

*Figure 35*

**Six Wire VAD System with Dedicated Bearing Coil and Fail-Safe Motor Operation**

*Figure 36*

**Four Wire VAD System with Dedicated Bearing Coil and Independent Motor and Bearing Operation without Fail-Safe**

*Figure 37*

**Seven Wire VAD System with Dedicated Bearing Coil and Fail-Safe Motor Operation and Independent Control**

Figure 38

Figure 39

a)

(b)

201

206

203

202

205

204

b)

206

205

201

Passive Bearing Magnet

Active Bearing Magnet

Active Bearing Winding

Motor Magnet

Motor Winding

Iron

Titanium

⊙ ◄ ⊗  Material Direction

Figure 40

300

306

303

305

304

301

302    307

Passive Bearing Magnet

Active Bearing Magnet

Active Bearing Winding

Motor Magnet

Motor Winding

Iron

Titanium

Figure 41

404
405

401
402
403

Passive Bearing Magnet

Motor Magnet

Motor Winding

Iron

Titanium

*Figure 42*

502   501

503

- Passive Bearing Magnet
- Motor Magnet
- Motor Winding
- Titanium

*Figure 43*

603
604

605

606

602
601

- Passive Bearing Magnet
- Active Bearing Magnet
- Active Bearing Winding
- Motor Magnet
- Motor Winding
- Iron
- Titanium

⊙ ◄ ⊗ Material Direction

Figure 44

700

701

702

Passive Bearing Magnet

Active Bearing Magnet

Active Bearing Winding

Motor Magnet

Motor Winding

Iron

Titanium

⊙ ◄ ⊗ Material Direction

Europäisches
Patentamt

European
Patent Office

Office européen
des brevets

# EUROPEAN SEARCH REPORT

Application Number

EP 19 21 1940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/314597 A1 (ALLAIRE PAUL E [US] ET AL) 23 October 2014 (2014-10-23) * paragraphs [0061] - [0069]; figures 13,15 * ----- | 1-5,8-13 | INV. A61M1/10 A61M1/12 |
| X | US 6 100 618 A (SCHOEB RETO [CH] ET AL) 8 August 2000 (2000-08-08) * the whole document * * ".. determine the position of the integral rotor 14 by determining the electrical impedance of the sensor windings."; column 16, lines 17-27 * ----- | 1-5,8-13 | |
| X | US 2016/144092 A1 (CASAS FERNANDO [US] ET AL) 26 May 2016 (2016-05-26) * the whole document * * In effect, the coils of the pump act as the transducer to measure BEMF and thus measuring displacement of the rotor and, indirectly, measuring thrust on the rotor.; paragraphs [0036], [0072] * ----- | 1-4,6-12 | |
| X | US 2009/118567 A1 (SIESS THORSTEN [DE]) 7 May 2009 (2009-05-07) * ".. axial position of the impeller may be determined by measuring the back emf .."; paragraph [0024] * ----- | 1-4,6-12 | TECHNICAL FIELDS SEARCHED (IPC) A61M |
| X | US 6 527 699 B1 (GOLDOWSKY MICHAEL P [US]) 4 March 2003 (2003-03-04) * column 7, lines 31-37 * ----- | 1-4,6-12 | |

The present search report has been drawn up for all claims

| Place of search | Date of completion of the search | Examiner |
|---|---|---|
| The Hague | 8 May 2020 | Van Veen, Jennifer |

## ANNEX TO THE EUROPEAN SEARCH REPORT
## ON EUROPEAN PATENT APPLICATION NO.

EP 19 21 1940

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2020

| Patent document cited in search report | | Publication date | Patent family member(s) | | Publication date |
|---|---|---|---|---|---|
| US 2014314597 | A1 | 23-10-2014 | AU | 741418 B2 | 29-11-2001 |
| | | | CA | 2330048 A1 | 28-10-1999 |
| | | | EP | 1073844 A2 | 07-02-2001 |
| | | | JP | 2002512333 A | 23-04-2002 |
| | | | KR | 20010071171 A | 28-07-2001 |
| | | | US | 7462019 B1 | 09-12-2008 |
| | | | US | 2008240947 A1 | 02-10-2008 |
| | | | US | 2014314597 A1 | 23-10-2014 |
| | | | WO | 9953974 A2 | 28-10-1999 |
| US 6100618 | A | 08-08-2000 | CA | 2210762 A1 | 10-10-1996 |
| | | | DE | 59607047 D1 | 12-07-2001 |
| | | | EP | 0819330 A1 | 21-01-1998 |
| | | | JP | 4076581 B2 | 16-04-2008 |
| | | | JP | H11503210 A | 23-03-1999 |
| | | | US | 6100618 A | 08-08-2000 |
| | | | WO | 9631934 A1 | 10-10-1996 |
| US 2016144092 | A1 | 26-05-2016 | CN | 107614029 A | 19-01-2018 |
| | | | EP | 3223879 A1 | 04-10-2017 |
| | | | JP | 2017535368 A | 30-11-2017 |
| | | | US | 2016144092 A1 | 26-05-2016 |
| | | | US | 2017333610 A1 | 23-11-2017 |
| | | | US | 2019001036 A1 | 03-01-2019 |
| | | | WO | 2016085985 A1 | 02-06-2016 |
| US 2009118567 | A1 | 07-05-2009 | CA | 2704196 A1 | 07-05-2009 |
| | | | CN | 101873870 A | 27-10-2010 |
| | | | CN | 105363081 A | 02-03-2016 |
| | | | EP | 2217302 A1 | 18-08-2010 |
| | | | EP | 3061472 A1 | 31-08-2016 |
| | | | EP | 3345635 A1 | 11-07-2018 |
| | | | ES | 2674388 T3 | 29-06-2018 |
| | | | JP | 5511672 B2 | 04-06-2014 |
| | | | JP | 2011503410 A | 27-01-2011 |
| | | | US | 2009118567 A1 | 07-05-2009 |
| | | | US | 2016045653 A1 | 18-02-2016 |
| | | | US | 2017043074 A1 | 16-02-2017 |
| | | | WO | 2009058726 A1 | 07-05-2009 |
| US 6527699 | B1 | 04-03-2003 | NONE | | |

EPO FORM P0459

For more details about this annex : see Official Journal of the European Patent Office, No. 12/82

**EP 3 827 852 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160281728 A1
- US 5588812 A
- US 006071093 A
- US 6302661 B1
- US 6071093 A
- EP 19159286
- WO 2018206754 A1